# EUROPEAN PATENT SPECIFICATION

(11) **EP 2 734 509 B1**
(45) Date of publication and mention of the grant of the patent: **26.12.2018**
(21) Application number: 12815444.0
(22) Date of filing: 09.07.2012
(51) Int. Cl.: C07D 307/33, C07D 207/267, C07C 51/41, C07C 51/42, C07C 55/02, C07C 55/10, C07C 57/15, C07C 59/245, C12P 7/46

(54) **METHODS FOR PREPARATION OF AMMONIUM SALTS OF C4 DIACIDS BY FERMENTATION AND INTEGRATED METHODS FOR MAKING C4 DERIVATIVES THEREOF**
VERFAHREN ZUR HERSTELLUNG VON AMMONIUMSALZEN AUS C4-DISÄUREN DURCH FERMENTATION UND INTEGRIERTE VERFAHREN ZUR HERSTELLUNG INTEGRIERTER C4-DERIVATE DAVON
MÉTHODES DE PRÉPARATION DE SELS D'AMMONIUM DE DIACIDES C4 PAR FERMENTATION ET MÉTHODES INTÉGRÉES DE FABRICATION DE DÉRIVÉS C4 DE CES DERNIERS

(30) Priority: 21.07.2011 US 201161510204 P; 21.07.2011 US 201161510209 P
(43) Date of publication of application: 28.05.2014
(73) Proprietor: Archer Daniels Midland Company, Decatur, IL 62526 (US)
(72) Inventor: MA, Chi-Cheng, Forsyth, Illinois 62535 (US); WERPY, Todd, Decatur, Illinois 62521 (US)
(74) Representative: dompatent von Kreisler Selting Werner - Partnerschaft von Patent- und Rechtsanwälten mbB
(86) International application number: PCT/US2012/045937
(87) International publication number: WO 2013/012590

(56) References cited:
- EP-A1- 1 686 183
- WO-A1-02/102772
- WO-A1-2009/024294
- WO-A1-2010/092155
- WO-A1-2011/123269
- WO-A2-2011/002895
- CA-A1- 2 625 511
- US-A- 2 816 923
- US-A- 3 113 138
- US-A- 5 008 468
- US-A- 5 101 045
- US-A- 5 969 164
- US-A1- 2010 044 626
- US-A1- 2010 094 051
- US-A1- 2011 245 514
- US-A1- 2011 245 515
- US-A1- 2012 015 411
- US-A1- 2012 095 241
- US-B1- 8 084 626
- US-B2- 6 603 021
- US-B2- 7 199 250
- US-B2- 8 193 375
- US-B2- 8 203 021
- CUKALOVIC ET AL.: 'Feasibility of production methods for succinic acid derivatives: a marriage of renewable resources and chemical technology' BIOPRODUCTS AND BIOREFINING vol. 2, no. 6, November 2008, pages 505 - 529, XP008139686
- MCKINLAY ET AL.: 'Prospects for a bio-based succinate industry' APPLIED MICROBIOLOGY AND BIOTECHNOLOGY vol. 76, no. 4, 2007, pages 727 - 740, XP019538718
- BECHTHOLD ET AL.: 'Succinic Acid: A New Platform Chemical for Biobased Polymers from Renewable Resources' CHEMICAL ENGINNERING & TECHNOLOGY vol. 31, no. 5, May 2008, pages 647 - 654, XP055014231
- KURZROCK ET AL.: 'Recovery of succinic acid from fermentation broth' BIOTECHNOLOGY LETTERS vol. 32, no. 3, 2010, pages 331 - 339, XP019787043

## Description

### BACKGROUND OF THE INVENTION

Ordinarily, the production of diacids such as succinic, malic, maleic and fumaric acid by fermentation of sugar by a microorganism involves recovery the diacid from the fermentation broth by formation of the calcium salt of the diacid, which is not soluble in the aqueous broth. In the case of fermentation by fungi such as *Rhizopus oryzae* or *Aspergillus oryzae,* which preferentially make fumaric and malic acid, respectively, the calcium is typically introduced into the broth in the form of CaCO₃, which forms Ca(HCO₃)₂ in solution. The bicarbonate is effective to maintain the pH of the broth as the diacid being produced tends to lower the pH. The diacid is recovered as the calcium salt form. The calcium salts of such C4 diacids have a very low solubility in aqueous solutions (typically less than 3 g/liter at room temperature), and are not suitable for many applications for which the free acid is needed, such as chemical conversion to derivative products like butanediol and the like. Therefore, the calcium salt is typically dissolved in sulfuric acid, forming insoluble calcium sulfate, which can readily be separated from the free diacid. Calcium sulfate is a product having few commercial applications, and accordingly is typically discarded as a solid waste in landfills or other solid waste disposal sites.

In an alternative process described for example in WO2010/147920, instead of using calcium carbonate, the pH of the medium for fungi growth was maintained using a magnesium oxygen containing compound, such as MgO, Mg(OH)₂, MgCO₃, or Mg(HCO₃)₂, all of which form the bicarbonate salt in aqueous solution. The use of magnesium rather than calcium was found to enhance production of the acid by fermentation. The fermentation was conducted at a pH of 5-8 and more preferably 6.0-7.0. The pH was maintained by the addition of the magnesium oxygen compound, and CO₂ was introduced into the medium in combination with the magnesium oxygen compound to maintain a molar fraction of bicarbonate (HCO₃⁻) of at least 0.1 and most preferably at least 0.3 based on the total moles of HCO₃⁻, CO₃⁻², and CO₂ in the medium. At the end of the fermentation, the liquid portion of the medium contained a majority of diacid as a soluble magnesium salt, which was separated from a solids portion of the medium containing precipitated salts and other insoluble material. The dissolved acid salt was converted into the free acid form by reducing the pH to below the isoelectric point of the diacid using a mineral acid such as sulfuric acid, and lowering the temperature of the medium to (most preferably) not greater than 5°C, which precipitated the free acid from the solution.

While useful for producing a free acid, the techniques described for using the magnesium salts results are expensive, first because the magnesium oxygen compounds cost considerably more than the analogous calcium compounds and the bulk of the magnesium remains in the fermentation medium in the form of the magnesium salt of the inorganic acid which is not economically useful for further fermentation or other purposes. Second, the need to lower the temperature of the recovered soluble salts to precipitate the free acid adds additional energy costs.

WO 2009/024294 and WO 2010/092155 disclose a method for preparing C₄ acid derivatives such as GBL, BDO and pyrrolidone compounds from succinic acid or ammonium salts thereof via a catalytic hydrogenation reaction.
The use of chromium chromite as catalyst is also disclosed.

WO 02/102772 discloses a method for preparing pyrrolidone derivatives like NMP from salts of succinic acid, in particular diammonium succinate, that can be obtained via fermentation. The method involves a catalytic hydrogenation step performed in the presence of methanol.

CA 2 625 511 discloses of method for preparing C₄ acid derivatives such as GBL and BDO from diammonium succinate prepared by fermentation via an esterification / hydrogenation sequence.

EP 1 686 183 discloses an organic acid ammonium solution produced by the steps of obtaining a fermentation broth containing organic acid magnesium by using an organic acid-producing microorganism in the presence of a magnesium compound, producing organic acid ammonium and a magnesium compound by subjecting the organic acid magnesium contained in the fermentation broth to salt-exchange using an ammonia compound, and separating the produced magnesium compound to obtain organic acid ammonium solution.

There is a need in the art therefore, to devise other methods for recovery of diacids from a fermentation media that will produce a diacid product suitable for use in subsequent chemical reactions, while also avoiding the production of calcium and/or magnesium waste products that contribute extra cost to the production of the diacids.

### SUMMARY OF THE INVENTION

In one aspect, described are methods of recovery of organic diacids from a fermentation process in a commercially useful form while reducing the accumulation of unusable waste products such as calcium sulfate. The method of recovery involves formation and separation of carbonate salts of the divalent metal cation of calcium, which are precipitated and filtered from a
fermentation broth while simultaneously forming ammonium salts of the diacid which remain soluble. The recovered metal carbonate precipitate can be reused in the fermentation process rather than discarded as unusable waste. The recovered filtrate containing the solubilized ammonium salts of the diacid can be subsequently processed into free diacids or directly used to make derivative products in single pot reactions or single pot reactions with and intervening removal of ammonium.

The present invention relates to a method of making a method of making C4 acid derivative compound, comprising, obtaining a clarified fermentation broth from growth of a microorganism to produce at least one diammonium salt of a C4 diacid selected from the group consisting of succinic, malic, maleic and fumaric acid and wherein the clarified fermentation broth containing the ammonium salt of a C4 diacid is obtained by:
- growing a microorganism in a fermentation media to produce a fermentation broth containing the C4 diacid;
- contacting the fermentation broth with the C4 diacid with a calcium compound to form a calcium salt of the diacid;
- contacting the fermentation broth with a source of ammonium and with a source of carbonate under a first condition including a temperature of at least 100°C and a pressure of at least 1480.28 kPa [200 psig] for a time sufficient to form a carbonate salt of at least 90% of the calcium and an ammonium salt of at least 90% of the C4 diacid in the fermentation broth;
- reducing the temperature and pressure of the contacted fermentation broth to a second condition effective to form a precipitate of the carbonate salt of calcium while maintaining the ammonium salt of the diacid in solution; and
- separating the precipitated carbonate salt of the calcium and cell mass in the fermentation broth from the solution of the ammonium salt of the diacid to form the clarified fermentation broth containing the ammonium salt of a C4 diacid; and
contacting the clarified fermentation broth containing the diammonium salt of the C4 diacid with a hydrogenation catalyst and H2 in a reaction mixture under conditions of temperature, pressure and time sufficient to hydrogenate the diammonium salt of the C4 diacid to form a derivative compound selected from the group consisting of gamma-butyrolactone (GBL) and 1,4-butane-diol (BDO).

Preferred embodiments are evident from the subject-matter of the dependent claims.

Also disclosed are integrated, single pot chemical hydrogenation methods for the synthesis of the commercially valuable solvent N-methyl-2-pyrrolidone (NMP) (not according to the invention) and the reagents gamma-butyrolactone (GBL) and 1,4-butane-diol (BDO) that are based on hydrogenation of the C4 diacids present in a fermentation broth, recovery of soluble ammonium salts thereof, and reduction of the ammonium salts or free diacids obtained therefrom to the desired compounds. The disclosure therefore provides a bio based alternative to NMP, GBL and BDO synthesis from renewable resources that does not rely on reagents produced from petrochemical sources.

### BRIEF DESCRIPTION OF THE DRAWINGS

Figure 1 illustrates an embodiment of a process for substituting partially soluble calcium diacid salts with soluble ammonium salts of the diacids forming insoluble calcium carbonate and the recycling thereof in a fermentation process in accordance with one aspect of the invention.
Figure 2 illustrates a reaction sequence for the production of NMP from ammonium succinate according to another aspect of the disclosure.
Figure 3 illustrates a reaction sequence for the reduction of mixed C4 diammonium diacids salts to ammonium succinate.
Figure 4 illustrates a reaction sequence for base catalyzed dehydration of malate to fumarate.
Figure 5a illustrates a reaction sequence for the production of 1,4 butanediol from mixed C4 diacids. Figure 5b illustrates production of dimethyl esters of C4 diacids in accordance with a reaction to produce BDO from fumarate or malate. Figure 5c illustrates direct reduction of succinic acid to butanediol.
Figure 6 illustrates a reaction sequence for the production of gamma butyrolactone from fumarate and succinate.
Figure 7 summarizes various embodiments of the combination of forming ammonium substitute salts from calcium or magnesium (not according to the invention) salts of C4 diacids with separation of the carbonates, and the hydrogenation thereof to form the derivatives 1,4 butanediol, N-methyl-2-pyrrolidone (not according to the invention), and gamma-butyrolactone.

### DETAILED DESCRIPTION OF THE INVENTION

**Production and Recovery of Ammonium Diacid Salts from Fermentation Media.** The present disclosure provides, in aspect, methods of production and recovery of organic diacids made by a fermentation process in the commercially useful form of ammonium salts, while reducing the accumulation of unusable waste products such as calcium sulfate or unusable forms of magnesium (not according to the invention).
The organic diacids most suitable for use in the methods of the present disclosure are the C4 diacids, succinic, malic, maleic, and fumaric acid. (Because maleic acid is the cis isomer of fumaric acid any statements herein regarding fumaric acid are equally applicable to maleic acid).

A variety of microorganisms can be used to produce diacids by fermentation. For the production of the C4 diacids, various species of the fungi *Asperigillus,* especially *A. flavus, A. oryzae,* and *A. sojae* are known to produce relatively high titers C4 diacids enriched with malic acid. Various species of the fungi *Rhizopus,* particularly *R. oryzae* are also known to produce relatively high titers of C4 diacids enriched with fumaric acid. Of these, the methods described herein have been employed for demonstrative, but not limited purposes, with fermentation media prepared from *A. oryzae* and *R. oryzae.*

Bacterial species are also known for production of C4 diacids, especially bacteria of various genera given a species designation "*succinogenes,* " which are so designated because they are known for producing diacids enriched in succinic acid. These include, for example, *Wolinella succinogenes, Fibrobacter succinogenes,* and *Actinobacillus succinogenes.* Of these, the methods described herein have been exemplified with *Actinobacillus succinogenes* fermentation media that produce a mixture of C4 diacids enriched with succinic acid.

Because all fermentation media, whether for fungi or bacterial fermentations contain similar compositions of nutrients in aqueous solution (*e.g*., a sugar carbon source, trace salts and vitamins along with divalent cations) and produce similar compositions of C4 diacids, the methods provided herein are applicable to any process that produces C4 diacids by fermentation by any microorganism even though the exact mixtures of C4 diacids produced may differ. Some fermentations, for example, fermentations with *Rhizopus* or *Asperigillus* produce small amounts of unwanted by-products such as acetic acid, glycerol, glutaric acid, ethanol and citric acid, however, these by product materials do not interfere with the recovery of the ammonium C4 diacid product or with direct use of the whole recovered clarified medium as a feedstock reagent for subsequent reactions.

Advantageously, the techniques described herein can be practiced for recovery of diacids from whole fermentation media (not according to the invention), clarified fermentation media, and purified fermentation media. "Whole fermentation media "means the complete fermentation broth inclusive of cell biomass and constituent nutrients, supplements and fermentation by-products. Example 4 shows that such a whole fermentation broth can be processed to convert a mixture of malate, fumarate and succinate salts to a mixture that has converted at least 96% of the malate and fumarate to diammonium succinate in a single pot series of steps. It is preferable to use whole fermentation media for reasons of cost and yield because the precipitated divalent metal carbonate salt is solid material that is difficult to separate from the particulate biomass of the fermentation media. "Clarified fermentation media," is the liquid fraction of crude fermentation media remaining after cell biomass and other suspended solids have been removed by filtration, centrifugation or other suitable technique. Example 3 shows recovery of ammonium diacids from a clarified fermentation media. "Purified" fermentation media is a clarified fermentation media that has been subject to at least one step to separate an unwanted component containing fraction from a fraction enriched with diacids. Typical techniques that may be used to obtain a purified fermentation media include, for example, distillation, ion exchange chromatography, electrodialysis, electrodeionization and ultrafiltration.

The methods rely in first part on introduction of sufficient carbonate into the fermentation under a first set of conditions of high temperature and pressure to form a partially insoluble carbonate salt of a divalent metal cation freeing the divalent metal from the diacid. In second part the method relies on the simultaneous formation of an ammonium salt of the diacid which is more soluble than the divalent metal salt of the diacid and much more soluble than the divalent metal carbonate salt. The ammonium salt will dissolve precipitated divalent metal salts of the diacids. Temperature and pressure conditions are then lowered to a second condition (typically standard temperature pressure (STP) *i.e*. 25°C, 101.35 kPa (14.7 psi), conditions is sufficient) whereby the metal carbonate salt quantitatively precipitates from the media leaving behind a solubilized fraction containing the ammonium salt of the diacid. The solubilized fraction is separated from the precipitated fraction by filtration or other means, and can be used directly as a reagent feedstock to form derivative products of the C4 diacids.

While the methods are most suitable for the C4 diacids (and only these are according to the invention) soluble salt is an ammonium salt, the methods are also applicable to separation of any organic acid or diacid produced by fermentation using a substitute salt, where a first physical conditions such as temperature and pressure can be applied so that (i) the carbonate salt of the divalent metal ion is less soluble in an aqueous medium than the corresponding diacid salt of the same metal cation; (ii) the substitute salt of the acid or diacid is at least 10 times more soluble than the divalent metal salt of the diacid under the first conditions; and (iii) the substitute salt of the diacid remains soluble under a second set of conditions where the carbonate salt of the divalent metal cation is insoluble.

Suitable divalent metal cations for the process include any where the carbonate salt thereof has a solubility in water of less than 0.5 g per liter at 25°C and a pH of 2 to 4. In preferred practices, the most suitable divalent metal cation is either calcium or magnesium whose carbonate salts have a solubility of approximately 0.02 and 0.4 g/l, respectively.
Only calcium is in accordance with the present invention.
Other functional divalent metal cations may include manganese, iron, cobalt, nickel, copper and zinc. Other functional, but less suitable divalent metal cations may include molybdenum, silver and cadmium. Calcium and magnesium (not according to the invention) are preferred because of their abundance and their particular suitability for use in alkaline forms as pH control supplements in a fermentation media that is used to produce the diacid in the first place. Moreover, carbonate salts of calcium and magnesium (not according to the invention) are alkaline and/or can readily be converted to other alkaline forms for re-use in pH control of the fermentation.

In typical practices the amount of divalent metal cation recovered from the fermentation medium is at least 90 % of the divalent metal cation that otherwise would form a salt of the diacid while the amount of recovered ammonium salt of the diacid is at least 90% of the amount of diacid present in the fermentation medium. The recovered metal carbonate salt can subsequently be converted into a soluble alkaline compound of the metal that can be recycled for continued use in the diacid production process by introduction into a new fermentation media to counterbalance the lowering of the pH that occurs during the production of the diacid.

In the methods provided herein, separation of diacids from a fermentation medium relies, in part, on the fact that under a first condition where a source of carbonate is infused into an aqueous media at elevated temperature and pressure a divalent metal cation that otherwise would form a partially or completely insoluble salt of the diacid preferentially complexes with the carbonate to form the divalent metal carbonate salt, while the diacid forms a salt of a substitute cation that remains soluble in the aqueous medium under the first condition. This first condition occurs at a temperature of at least 100°C and a pressure of at least 1480.28 kPa (200 psig). In one exemplary practice the temperature was 120°C and the pressure was 1480.28 to 1687.12 kPa (200-230 psig). Yet higher temperatures improve solubility of substitute salts of the diacid and of CO₂ without substantially increasing of the solubility of the divalent metal carbonate that is formed. In certain exemplary practices, a temperature range of 120°C - 230°C was used and the carbonate was infused by introducing CO₂ at a pressure of 1480.28 to 3548.70 kPa (200- 500 psig).

In one step, carbonate is introduced into a fermentation medium containing the diacid. The most effective way to introduce the carbonate is by infusion with CO₂ under pressure of at least 1480.28 kPa (200 psig) at a temperature of at least 120°C. Carbonate can also be introduced by using a partially solubilized carbonate slurry suspension that will further dissolve upon dilution into the medium or by use of a solubilized bicarbonate salt at a pH that will form the carbonate. For example, magnesium or calcium bicarbonate solutions or NH₄HCO₃, Na₂CO₃, or NaHCO₃ with the medium at a pH of greater than 6 may also be used. The amount of carbonate to introduce should at least be one molar equivalent to the amount of divalent metal cation that is present in the media at the time it is desired to recover the diacids produced. More typically, the amount of carbonate should be between one and two molar equivalents of the amount of divalent metal cation.

The amount of divalent metal cation in turn will be predicated on the amount of diacid produced, or expected to be produced, by the fermentation process. Typically, the amount of divalent metal cation should be about one half to two molar equivalents of the amount of diacid produced or expected to be produced. In exemplary practices the amount of divalent metal cation used was 1.2 to 1.6 molar equivalents to the amount of diacid produced. It is preferable to introduce the divalent metal cation as a soluble salt, for example as calcium bicarbonate, or magnesium sulfate. Only salts of calcium are in accordance with the invention. Some divalent metal cation salts of calcium and magnesium, however, such as calcium carbonate,
magnesium carbonate, and magnesium hydroxide are only partially soluble at neutral pH. These materials may be introduced into the medium as partly solubilized slurry in water or as a dry material that will dissolve when diluted into the larger volume of the fermentation medium at the appropriate pH.

For example, in one exemplary practice, when Mg(OH)₂ (not according to the invention) was used to control the pH of fermentation to produce succinic acid from
*Actinobacillus succinogenes,* when the fermentation media began to dip below pH 6.9, a slurry of Mg(OH)₂ was added to adjust the pH with the total amount of magnesium added by the end of fermentation being about 1.6 molar equivalents of the amount of succinate produced. In other exemplary practices using *Rhizopus oryzae* to produce primarily fumarate at an optimal pH of about 5.8, or using *Asperigillus oryzae* to produce primarily malate at an optimal pH of between 6 and 7, a slurry of CaCO₃ was added to the fermentation media when the pH began to lower below these optimal ranges, with the total amount of calcium added by the end of fermentation being about 1.2 to 1.3 molar equivalents of the amount of the total diacids produced.

Depending on the tolerance of the diacid producing organism to low pH, the divalent metal cation can be introduced into the medium before, during or after the fermentation process that produces the diacid.
In the present invention, the fermentation broth containing the C4 diacid is contracted with the calcium compound to form a calcium salt of the diacid.
If the diacid producing organism has high tolerance to low pH so that production of the diacid does not inhibit fermentation by the microorganism, the divalent metal cation can be introduced after the fermentation is complete. In this case the divalent metal cation can be introduced in any suitable salt form or as an oxide. Suitable salt forms include the carbonate, bicarbonate, hydroxide or halide salts of the divalent metal cation. If the diacid producing microorganisms is inhibited in the fermentation process by low pH, then preferably the divalent
metal cation is introduced as an alkaline compound such as in the oxide form or as the bicarbonate or carbonate salt continuously or intermittently during the fermentation process to counter the lowering of pH as mentioned above. If pH control is not important to fermentation yield, then the divalent metal cation may be introduced at any time before, during or after the fermentation process and in any salt form or alkaline form.

In addition to infusing carbonate into the medium, ammonium as the substitute cation for formation of the diacid salt is also introduced into the medium and these conditions are maintained for long enough to equilibrate the formation of soluble ammonium salt of the diacid and the divalent salt of the carbonate. As used herein, "ammonium salt of the diacid" or simply "ammonium diacid" means at least one of a mono ammonium salt of the diacid having one free acid group and one ammonium group, or a diammonium salt of both acid groups. It is important to note that the time for equilibration includes time needed to redissolve divalent cation salts of the diacids that have previously formed and begun to precipitate from the media and to substitute the ammonium ion for the diacid which will maintain the solubility thereof. In exemplary practices, the conditions used for formation of divalent metal cation and substitution to make the diammonium salt of the diacid, were a temperature of at least 100°C, a pressure of at least 1480.28 kPa (200 psig), and a pH of 8 to 11. In particular exemplary practices the temperature was 120°C to 230°C, the pressure was 1480.28 to 3548.70 kPa (200 psig to 500 psig), the pH was 8-9, and the time for equilibration
under these conditions was about 2 hours.

Ammonia, or any ammonium donating salt may be used in the method, including organic or inorganic ammonium salts. For reasons of subsequent possessing and derivatization of the diacid, it is preferred to use an inorganic ammonium salt, such as ammonium hydroxide, ammonium sulfate or an ammonium halide. It is most preferable to use ammonium hydroxide so as not to introduce any other ions other than H⁺ and -OH, or introduce other chemically reactive functional groups such as sulfate if it is desired to further perform single pot reactions as described herein after. The amount of ammonium salt will depend on the amount of diacid in the recovered media and the type of ammonium salt desired. If the mono ammonium salt is desired, the amount of ammonium should be about one molar equivalent to the amount of diacid. If the diammonium salt is desired, the amount of ammonium should be at least two molar equivalents to the amount of diacid present. In exemplary practices for forming the diammonium salts of the diacids, 3-4 molar equivalents of ammonium hydroxide was used.

After equilibration under the first condition is reached, the pressure is released and the temperature is lowered to ambient temperature providing a second condition whereby the divalent metal carbonate salt will quantitatively precipitate from the fermentation media while the ammonium salt remains solubilized. In the case of calcium or magnesium (not according to the invention) the second
condition can be a temperature at least as high as room temperature (25°C), however, depending on concentration of the salts; higher temperatures may also work with prolonged incubation. Temperatures lower than room temperature will also work, provided the temperature is not so low as to cause precipitation of ammonium salt of the diacid, and provided there is not such an excess of the divalent metal cation relative to the ammonium that formation of greater than 10% of the metal salt of the diacid also occurs where the temperature is such that the metal salt of the diacid also precipitates, lowering recovery of the diacid in the form of the soluble ammonium salt.

The precipitated divalent metal carbonate salt is separated from the fermentation medium by any suitable means known in the art such as filtration or centrifugation. In certain embodiments of the method, the separated metal carbonate salt is recovered and either reused as is, or converted to another alkaline form for recycled use in the fermentation process. For example, in one alternative, a slurry of the recovered calcium carbonate can be directly added to a new acidic fermentation media in part dissolving into calcium bicarbonate to raise the pH. In another alternative, the recovered calcium carbonate can first be dissolved in a mineral acid forming calcium bicarbonate directly which can also be used to adjust pH in solution form. In yet another practice, the calcium carbonate can be decomposed to the compound calcium oxide by heating at a temperature of 825°C or higher, which will liberate CO₂ that can be recaptured by compression. The analogous reaction also occurs with magnesium carbonate (MgCO₃) which decompose to MgO at even lower temperatures in the range of 250 °C - 800C, with the typical temperature for 100% conversion being about 500°C - 662°C. Calcium oxide and magnesium oxide both convert to their respective hydroxide compounds when dissolved in aqueous media, providing an alternative alkaline compound that can be recycled to the fermentation media for pH control. As yet another alternative practice, magnesium carbonate can also be converted to its water soluble bicarbonate Mg(HCO₃)₂ by treatment with acid, and the alkaline bicarbonate used to adjust the pH of the fermentation medium.

While it is most desirable to recycle the recovered divalent alkaline compound by use in subsequent rounds of fermentation, as another option, the metal carbonate salt or its alkaline derivatives can be sold for use in other processes, such as for making building materials.

The filtrate or supernatant depleted of the metal carbonate and containing the solubilized ammonium salt of the diacid is also recovered. This ammonium diacid containing fraction can be used directly for further conversion to other compounds, for example by the techniques described hereafter, or the free diacid or ammonium diacid salt can be further purified. For example, the free acid can be generated by acidifying the media to form the free diacid. The free diacid then can readily be separated from the ammonium ion by ion exchange chromatography or other conventional ion removal process such as electro deionization or electrodialysis. In one practice, the recovered filtrate is concentrated by evaporation into a concentrated liquid or solid product that directly used as a reagent feedstock for further processing.

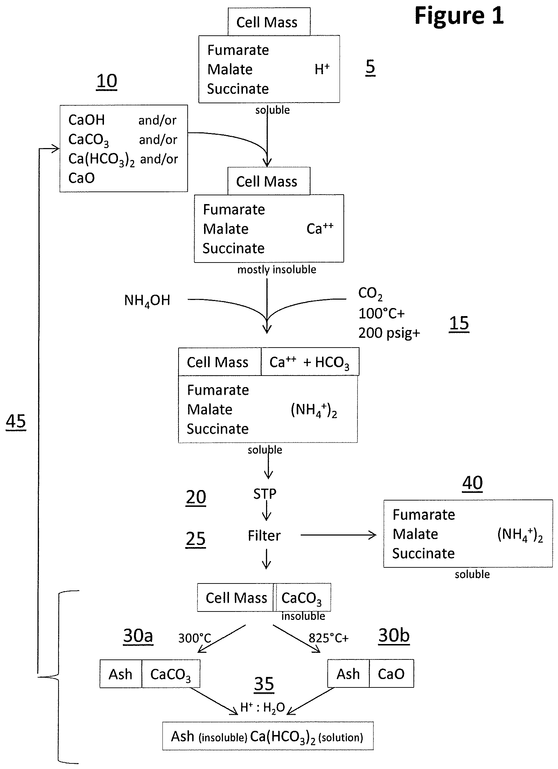

Reference is made to Figure 1 that depicts an exemplary embodiment of a fermentation process for production of one or more of the C4 diacids where a soluble ammonium salt of the diacids is formed with the simultaneous formation of an insoluble calcium carbonate or other alkaline derivatives calcium oxide and/or calcium bicarbonate that may be recycled to control the pH of an ongoing fermentation. Depending in the microorganism, the pH should typically be maintained between 5.5 and 7.5. During the early stages of fermentation 5 in a nutritive fermentation broth, cell mass is produced along with the free C4 diacids, fumaric, malic and/or succinic. The free C4 diacids lower the pH of the fermentation media, which is countered at step 10 by introduction of one or more of the alkaline forms of oxy calcium compounds - calcium hydroxide, calcium carbonate, calcium oxide and/or calcium bicarbonate. The introduced oxy calcium compound forms partially insoluble calcium salts of the C4 diacids. At the conclusion of the fermentation at step 15 ammonium hydroxide is added to the fermentation media along with infusion of carbon dioxide at a temperature of at least 100°C and a pressure of at least 1480.28 kPa (200 psig) initially forming soluble calcium bicarbonate and ammonium salts of
the diacids. Instead of carbon dioxide, the media could also be infused with another salt of bicarbonate, such as sodium bicarbonate, or more preferably ammonium bicarbonate. The mixture is allowed to incubate at the elevated temperature and pressure conditions long enough to quantitatively substitute ammonium for the calcium salts of the diacids, including the fraction partially precipitated, and to form calcium carbonate. At step 20 the mixture is returned to ambient temperature and pressure conditions (*e.g*., STP), which results in quantitative formation of insoluble calcium carbonate. At step 25 the precipitated calcium carbonate is separated along with the cell mass by filtration or centrifugation and the soluble ammonium salts of the diacids are recovered 40 in the filtrate or supernatant. At step 30a or 30b the recovered calcium carbonate in the retentate or pellet, along with the cell mass is heated to a temperature and for a time sufficient to convert the cell mass into ash. The cellular ash contains trace minerals that are useful supplements to promote new cellular growth in the fermentation media.

In the alternative step 30a using temperature of about 300°C for about 2 hours the cell mass is converted into an insoluble ash and the calcium compound is in the dried calcium carbonate form as solid materials. In the alternative step 30b using a temperature of at least 825°C for about 1 hour the calcium carbonate is decomposed into dried calcium oxide solid material with the liberation of carbon dioxide. As an option, at step 35 the calcium carbonate or the calcium oxide can be dissolved in a mineral acid such as HCl forming a solution of calcium bicarbonate and insoluble ash, which if desired, can be separated by filtration. At step 45 any of the recovered oxy calcium materials with or without the ash may be recycled to adjust the pH of fermentation at step 10. In an exemplary practice for production of mixed C4 diacids (fumarate, malate, succinate) by fermentation with *Rhizopus oryzae,* a slurry of calcium carbonate was used as the pH adjusting compound at step 10.

While illustrated with oxy calcium compounds in Figure 1, the process is essentially identical when using the analogous oxy magnesium compounds magnesium carbonate, magnesium oxide, magnesium hydroxide or magnesium oxide (which forms magnesium hydroxide in aqueous solutions). In an exemplary practice for production of primarily succinate by fermentation with *Actinobacillus succinogenes,* a slurry of magnesium hydroxide was used as the pH adjusting compound at step 10.

Single Pot Reduction of Ammonium Diacids from Fermentation Media. In a further aspect of this disclosure (not according to the invention), the recovered ammonium salts of the C4 diacids produced in a fermentation media are used as an alternative source for making the widely used solvent and reagent N-methyl-2-pyrrolidone (NMP).

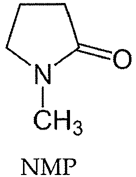

NMP and its derivatives are used as intermediates for the synthesis of agrochemicals, pharmaceuticals, textile auxiliaries, plasticizers, stabilizers and specialty inks. It is also employed as a nylon precursor. To make NMP from the ammonium diacid is a one two, three or four step process depending on the diacid moiety, all of which can be conducted in a single reaction vessel without intervening purification of intermediates.

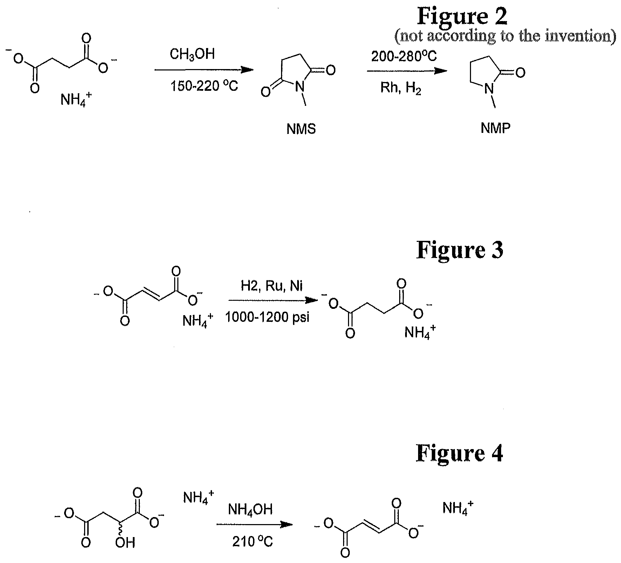

In the case of ammonium succinate, NMP is made in a one or two step process, that includes combining the ammonium succinate with a molar excess of methanol and hydrogen to form a reaction mixture and heating the reaction mixture to a temperature of 200°C to 300°C, most typically about 230°C, in the presence of a first hydrogenation catalyst for time sufficient to initially form the cyclic diamide N methyl succinamide (NMS, *aka.* 1-methyl-2,5-pyrrolidinedione). With prolonged incubation times, the NMS is further hydrogenated to NMP according to the reaction sequence illustrated in Figure 2. The hydrogenation steps can be done in single step using a single catalyst. Alternatively, the reaction may be done sequentially in a two step process, where a first hydrogenation catalyst is used under a first set of conditions to produce NMS and a second hydrogenation catalyst is used under a second set of temperature conditions to produce the NMP.

In the case of ammonium fumarate (or maleate), NMP is made in a two step process that includes a first hydrogenation step with a first hydrogenation catalyst to reduce the double bond of the fumarate prior to introduction of methanol followed by a second hydrogenation step in the presence of a second hydrogenation catalyst and methanol as shown in Figure 3.

In the case of ammonium malate, NMP is made in a three step process that includes a prior dehydration of the hydoxy group of malate by merely heating the ammonium malate in aqueous solution to a temperature of at least 210°C, which can be done in the absence of a hydrogenation catalyst to form ammonium fumarate in a sequence depicted by Figure 4. While the initial dehydration may be performed in the absence of the hydrogenation catalyst, the catalysts may optionally be included in the initial dehydration step without detriment to the reaction sequence.

Suitable catalysts for the hydrogenation reactions in any of the foregoing steps include, but are not limited to nickel, (*e.g*,, Raney nickel, G-49B available from Sud Chemie (Louisville, KY) which is nickel on kiselghur with a zirconium promoter), ruthenium, e.g. Ru/C, which is ruthenium on a carbons substrate), palladium as in for example palladium on carbon (Pd/C), or copper chromite (Ru/C, Pd/C, Pt/C). It is preferred to use a ruthenium and/or rhodium catalyst for single catalyst hydrogenation reactions. When nickel is used in a multi catalyst hydrogenation sequence, the nickel catalyst is preferably used as the first hydrogenations catalyst for the reduction of the C4 acids to succinate, and the ruthenium and/or rhodium is used for subsequent hydrogenations to produce NMP in the presence of methanol. The hydrogenation reactions typically require infusion under a H₂ atmosphere at a pressure of at least 790.80 kPa (100 psig), most typically between 1480.28 to 3548.70 kPa (200 - 500 psig) and at temperatures of greater than 100°C, typically between 120-300°C for a time
sufficient to hydrogenate (reduce) the relevant bonds.

In one embodiment, the ammonium salts of the diacids are used as an alternative source of making the solvent and reagent 1,4 butanediol (BDO). 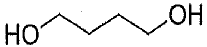 BDO can be made by alternative routes, depending on the starting C4 diacid.

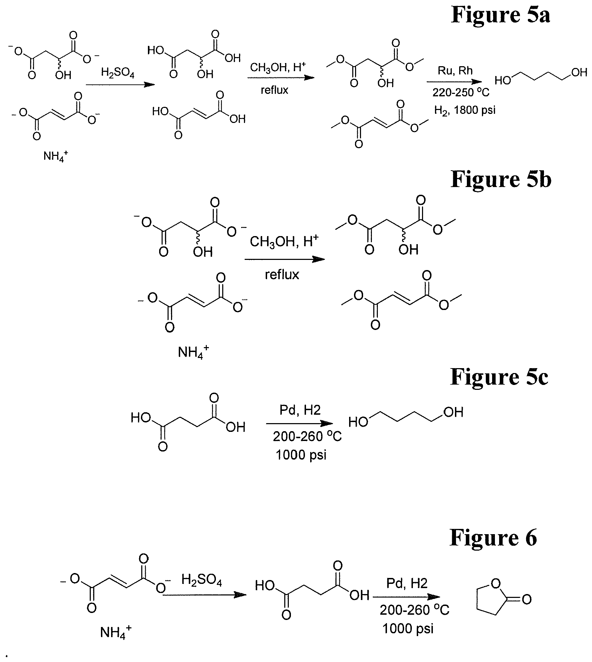

When the starting material is predominantly ammonium fumarate and/or ammonium maleate, there are two routes to BDO, each of which requires separation of ammonium from the salt. A first route includes the steps of (i) acidifying the reaction mixture to form the free acid and ammonium, (ii), removing the ammonium by ion exchange, electrodialysis, electrodeionization or other suitable ion removal technique; (ii) adding methanol and an acidic or basic catalyst to form the dimethyl fumaryl ester; and reducing the dimethyl fumarate to BDO by hydrogenation in the presence of a suitable hydrogenation catalyst as illustrated in Figure 5A. Suitable acid or base catalyst include simple homogenous mineral acids such as H₂SO4, HCl, and mineral bases such as NaOH, or strongly acidic or strongly basic heterogeneous catalyst such as sulfated or phosphated acidic ion exchange resins or basic ion exchange resins having amino or methoxy functional groups. Example conditions for the esterification reaction are to reflux the ammonium fumarate in 10% sulfuric acid for about 1 hour. Suitable hydrogenation catalyst and conditions for the conversion of the diester to BDO are the same as mentioned herein before with respect to the hydrogenation reactions for making NMP. Suitable hydrogenation catalysts and conditions for the conversion of the diester to BDO are the same as mentioned herein before with respect to the hydrogenation reactions for making NMP. The preferred catalyst for hydrogenation are Ni, Re, Rh, Ru, Pd, and Au.

A second route for BDO synthesis when the starting material is predominantly ammonium fumarate and/or ammonium maleate is to simultaneously conduct the anion exchange separation of the ammonium ion and methyl esterification of fumarate by contacting the ammonium fumarate with an acidic ion exchange resin over a column in the presence of methanol as illustrated in Figure 5B. In his case, the column will in-part function as an ion exchange column preferentially retaining ammonium to a portion of the acidic functionality, while the remainders of portion of acidic groups act as a catalyst to esterify the fumarate to the methanol. The dimethyl fumarcyl ester which elutes from the column is then subject to reduction in the presence of H₂, heat and a metal hydrogenation catalyst to make BDO as in the first route.

Of course, BDO can also be synthesized from ammonium malate using the same two routes mentioned above, except that prior to the ammonium separation or contact with methanol, the malate is converted to fumarate by heat catalyzed dehydration as mentioned herein before for the production of NMP.

The third route for synthesis of BDO can be used when the C4 diacid is primarily ammonium succinate. In this case there is no need to esterify the diacid to the dimethyl ester derivative. Instead, a mineral acid is added in sufficient amounts to form free succinic acid and.the ammonium is substituted with H⁺ by ion exchange, electrodialysis, electrodeionization or other suitable ion removal step, and the succinic acid is directly subject to reductive hydrogenation in the presence of hydrogen with a palladium and/or ruthenium catalyst to form BDO as illustrated in Figure 5C. Conditions for direct reduction of the diacid to the diol are the same as those required for reduction of the methyl diester to the diol.

In other embodiments, butyrolactone (GBL) can also be directly produced from the ammonium succinate. 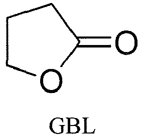 The route for production of GBL is illustrated in Figure 6. As with the production of BOD a mineral acid is added in sufficient amounts to form free succinic acid and the ammonium may optionally be removed by ion exchange or other suitable technique. The fee succinic acid is reduced to GBL by hydrogenation in the presence of a palladium/ Al₂O₃, catalyst, which may be palladium on carbon in the presence of Al₂O₃ or palladium on a Al₂O₃ support. A suitable solvent is dioxane, and a suitable temperature is about 280°C for 4 hours at a pressure of around 6 MPa (60 bars).

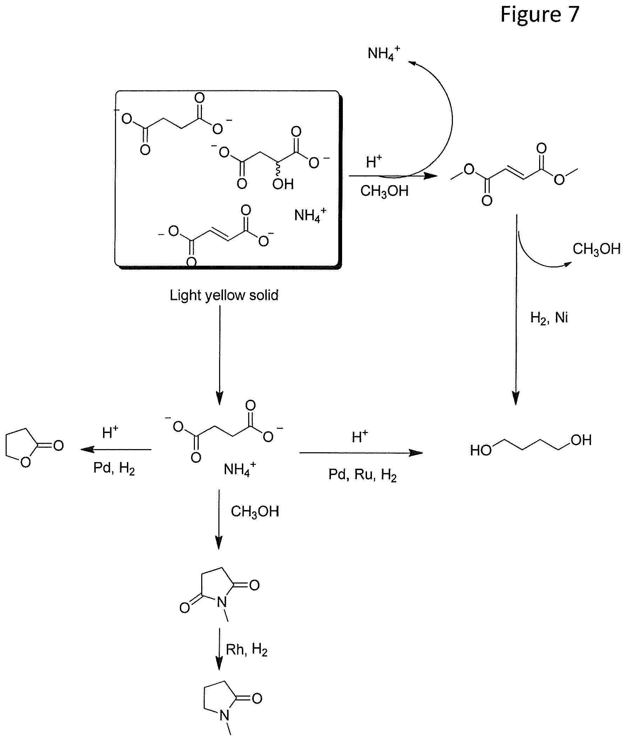

Figure 7 summarizes an integrated process of fermentation to produce C4 diacids, conversion of the divalent salts thereof to ammonium salts, and subsequent hydrogenation reactions to make a variety of reduced derivatives. Steps 5-30 of Figure 1 represented in the upper right of Figure 7 are performed in the fermentation process resulting in the recovery of a filtrate of solubilized ammonium diacids 50 in aqueous solution. The recovered filtrate may be used directly or optionally may be concentrated by evaporation. If the fermentation preferentially produces malate, as in the case of *A. oryzae* then at step 55 acid heterogeneous or homogeneous acid or base catalysis may be used to dehydrate the malate to fumarate with removal of the ammonium followed by esterification of the fumarate to form dimethyl fumarate 60. Dimethyl fumarate may then contacted with a hydrogenation catalyst in the presence of H₂ to form BDO. In alternative procedure for making BDO, the mixed ammonium salts of the diacids may be hydrogenated over a first catalyst to covert them all to diammonium succinic succinate and the ammonium exchanged with hydrogen by addition of an acid. The succinate can be further hydrogenated to form BDO with or without removal of the ammonium. The two step hydrogenation can be performed in a single pot using a single catalyst or a different catalyst may be used for the first and second hydrogenations in the reaction sequence. In an embodiment for making NMP (not according to the invention), the diammonium succinic acid formed in the fermentation broth can be mixed with methanol and
a hydrogenation catalyst to form NMS and then NMP in a two step reaction sequence that requires the presence of the ammonium. The two step reaction sequence can be performed in a single pot with a single hydrogenation catalyst, or different hydrogenation catalysts may be used for the first and second steps in the reaction sequence. In an embodiment for making GBL, similar to making BDO, ammonium succinate is converted to succinate by ion exchange with a hydrogen ion and the succinate is hydrogenated over a palladium catalyst to yield GBL.

The examples that follow are provided to illustrate various aspects of the invention.

### Example 1

### Separation of diammonium malate and calcium carbonate from calcium malate (dilute sample)

A mixture of 7.01 g (0.04 mol) of calcium malate and 20 mL of NH₄OH (28%) in 150 mL of water, was pressurized under 500 psi of CO₂ at room temperature. The reaction mixture was stirred for 2 h while incubated at 120°C. After the reaction, the gas was released and the mixture was cooled to room temperature and a white solid precipitate of calcium carbonate (5.64 g) was formed, which was collected by filtration and dried. The flow-through filtrate was evaporated under vacuum to obtain an oily product, which was ammonium malate (6.11 g). The yield of ammonium malate was 93%, based on the calcium malate.

### Example 2

### Separation of diammonium malate and calcium carbonate from calcium malate (concentrated sample)

A sample of 20.28 g (0.12 mol) calcium malate was mixed with 30 mL of NH₄OH (28%) in 200 mL of water and charged under 60 psi of CO₂ at room temperature. The mixture was stirred for 2 h while incubated at 120 °C and reached a pressure at 180 psi. After the reaction, the gas was released and the mixture was cooled to room temperature and the white solid was filtered out. The flow-through filtrate was evaporated under vacuum to obtain an oily product, which was determined to be ammonium malate (21.76 g). The filter cake was calcium carbonate (11.27 g). The yield of ammonium malate was 96%,based on the calcium malate input.

### Example 3

### Recovery of diammonium diacid salts from a fermentation media

A clarified fermentation broth of 502.82 g obtained by growing a *Rhizopus oryzae* on glucose to produce a mixture of fumaric, succinic and malic acid was mixed with 35 ml of NH₄OH (28%), and pressurized under 500 psi of CO₂ at room temperature. The mixture was stirred for 2 h while incubated at 120 °C. After the reaction, the gas was released and the mixture cooled to room temperature and the white solid precipitated (44.87 g) that formed was collected by filtration and determined to be primarily CaCO₃ and contained 0.5% of free malic acid and 0.8% of free fumaric acid. The flow-through filtrate was evaporated under vacuum to obtain 54.368 of a light brown solid product, which was determined to be a mixture of ammonium salts, including 65.6% ammonium fumarate, 18% ammonium malate and 15.8% diammonium succinate (DAS).

### Example 4 (formation of NMP is not according to the invention)

### Single pot removal of calcium carbonate, formation of mixed ammonium C4 diacids, reduction to succinate and formation of NMP

A whole broth from fermentation of glucose to form C4 diacids by *Rhizopus* according to Example 6 was obtained. The whole broth contained calcium salts of the diacid, unconsumed glucose, cell biomass and fermentation by-products such as glycerol and acetic acid. Based on analytical results, the whole broth contained 46.9 g/kg fumaric acid, 19.2g/kg malic acid and 3.3g/kg of succinic acid (calculated as free acids although present in the form of Ca salts in the broth).

The whole broth (393.87 g) was treated by addition of 73 ml of NH₄OH (28%) and infusion with CO₂ at 200 psi at a temperature of 80°C for 2 hours. A nickel catalyst in the form of Raney-nickel or a palladium catalyst on carbon (Pd/C) was added to certain samples of the ammonium treated and carbonated broth as indicated by the table below, which were then heated to a temperature of 120°C at a pressure of 500 psig of H₂ and stirred for a period of 1 hour. On sample did not contain any catalyst. Two samples were further heated to a temperature of 230°C and stirred for another 2 hours. In one of the 230°C samples 18 ml of methanol was also added to the hydrogenation mixture. Heating the whole broth under these conditions also affected a kill step on the *Rhizopus* biomass inactivating further biological processes.

The temperature was reduced to room temperature for a period of 2 hours, and the suspended solid material comprised of calcium carbonate salt and cell biomass was collected by filtration, and the filtrate that contained dissolved diammonium salts of the diacids was recovered and analyzed giving the results shown in Table 1 below:

**Table 1**

| temperature | Fermentation broth (g) | Diammonium Malate (g/l) | Diammonium Fumarate (g/l) | diammonium succinate (g/l) |
|---|---|---|---|---|
| 120 | 460.86 | 11.52 | 0.01 | 23.59 |
| 230** | 393.87 | 0.47 | 0.32 | 16.20 |
| 230* | 379.00 | 0.60 | 0.14 | 18.22 |
| 120 | 463.00 | 8.28 | 0.79 | 37.67 |

| | | | | |
|---|---|---|---|---|
| * methanol was added to the broth, catalyst was Ni. ** Pd/C was the catalyst | | | | |

Based on the content of the starting broth, the results indicated that in even in the absence of a catalyst and at the relatively low temperature of 120 °C, greater than 98% of the fumarate and at least and at least 60% of the malate in the medium was converted to diammonium succinate. In the presence of either metal catalyst at a temperature of 230 °C at least 95% of the combined fraction of malate and fumarate were converted into diammonium succinate. In the presence of methanol at 230°C a substantial portion of diacids were further reduced to NMS and NMP as shown in Table 2.

**Table 2**

| temperature | Malic acid | Succinic acid | Fumaric acid | NMP | NMS |
|---|---|---|---|---|---|
| 230 | 0.074 | 3.16 | 0.007 | 12.5 | 10.4 |

| | | | | | |
|---|---|---|---|---|---|
| g/kg | | | | | |

### Example 5 (formation of NMP is not according to the invention)

### Single pot conversion of ammonium C4 diacids to NMP

An evaporated broth from an *Actinobaccillus succinogenes* fermentation to produce C4 diacids that was converted into ammonium diacid salts from calcium salts was obtained. The evaporated broth, which contained 64% ammonium succinate, 25.4% malic acid, and 10.3% glycerol was mixed with 2.32 g of Raney Nickel and 25 ml of methanol in 200 ml water and heated to 280°C in an autoclave reactor fitted with temperature and pressure controllers. The air was removed by bubbling hydrogen three times through a dip-tube and the hydrogen was charged at 400 psig at room temperature. The mixture was heated to 280°C for 2 hours, during which time some hydrogen was consumed. The mixture was stirred for another 6 hours at 280°C with H₂ pressure at 1200 psig. Afterwards, the reactor was cooled to room temperature and the residual hydrogen released. The catalyst was filtered out, the recovered filtrate was evaporated under vacuum to obtain a white solid material that was analyzed and shown to contain 10.36 g/kg of NMS, 12.45 g/kg of NMP, and 3.16 g/kg of diammonium succinate.

### Example 6

### Fermentation media, conditions and yields of C4 diacids from Actinobaciilus succinogenes, Rhizopus oryzae, and Aspergillus oryzae

A. For *Actinobacillus succinogenes,* the strain was obtained from Michigan Biotechnology Institute (Lansing, MI). The media contained in g/l:

| | |
|---|---|
| Corn Steep Liquor | 20 |
| Betaine | 0.5 |
| Glutamic Acid | 0.5 |
| Biotin | 0.0002 |
| Na Phosphate Buffer (0.5M) | 7.0 ml/l |
| Na₂CO₃ (20% solution) | 2.6 ml/l |
| Dextrose | 137.5 |

Over the course of the fermentation the pH was adjusted with Mg(OH) totaling 87.5 g/l. The fermentation growth parameters were:

| | |
|---|---|
| pH | 6.9 |
| Temperature | 38C |
| Agitation | 250 rpm |
| CO₂ | 0.025 vvm |

The major byproducts of the fermentation were:

| | |
|---|---|
| Glycerol | <1 g/l |
| Acetate | 0-3g/l |
| Ethanol | < 1g/l |
| Pyruvate | 1-5 g/l |

The final titer, yield and productivity of C4 diacids was:

| | Succinic acid only |
|---|---|
| Titer (g/l) | 100g/l |
| Yield from dextrose | 90% |
| Productivity | 2.0 g/l/hr |

B. For *Rhizopus oryzae,* the strain was obtained from Archer Daniels Midland Company, Decatur Illinois. The media contained in g/l:

| | |
|---|---|
| Corn Steep Liquor | 0.19 (dry solids basis) |
| (NH₄)₂SO₄ | 1.35 |
| KH₂PO₄ | 0.225 |
| MgSO₄ * 7H20 | 0.30 |
| Trace Metals Solution | 7.5ml |

Composed of:

| | |
|---|---|
| ZnSO₄*7H₂O | 4.4 g |
| FeCl₃*6H₂O | 0.75 g |
| Tartaric Acid | 0.75 g |
| dH2O | 1000 ml |

Over the course of the fermentation the pH was adjusted with a slurry of CaCO₃ totaling 90 g/l. The fermentation growth parameters were:

| | |
|---|---|
| pH | 5.8 |
| Temperature | 34°C |
| Agitation | 200-500 rpm |
| Aeration | 0.05 vvm |

The major byproducts of the fermentation were:

| | |
|---|---|
| Glycerol | 10-25 g/l |
| Acetic Acid | <1 g/l |
| Ethanol | 1-10 g/l |
| 2-ketoglutaric acid | 1-5g/l |

The final titer, yield and productivity of C4 diacids was:

| | Fumaric | Malic | Succinic |
|---|---|---|---|
| Titer (g/l) | 30-50 | 5-30 | 2-10 |
| Yield from dextrose | 57% | | |
| Productivity | 1.81 g/l/hr | | |

C For *Aspergillus oryzae,* the strain was obtained from Novozymes (Washington, DC). The media contained in g/l:

| | |
|---|---|
| Bacto peptone | 9 |
| KH₂PO₄ | 0.15 |
| K₂HPO₄ | 0.15 |
| MgSO4 . 7H2O | 0.1 |
| CaCl₂ | 0.1 |
| FeSO₄.7H2O | 0.005 |
| NaCl | 0.005 |
| Biotin stock solution (5mM) | 1.0 ml |
| Pluronic L61 | 0.5 ml |
| Dextrose | 198 |

Over the course of the fermentation the pH was adjusted with CaCO₃ totaling 120 g/l. The fermentation growth parameters were:

| | |
|---|---|
| pH | 6 - 7 |
| Temperature | 34 °C |
| Agitation | 300-700 rpm |
| Aeration | 1.0 vvm |

The major byproducts of the fermentation were:

| | |
|---|---|
| Glycerol | <1g/l |
| Acetate | 0<1g/l |
| Ethanol | 0-2 g/l |
| Citric Acid | 1-4 g/l |

The final titer, yield and productivity of C4 diacids was:

| | Malic | Succinic |
|---|---|---|
| Titer (g/l) | 141.5 | 11.2 |
| Yield from dextrose | 80% | 7% |
| Productivity (g/l/hr) 1 | .11 | 0.9 |

## Claims

1. A method of making C4 acid derivative compound, comprising,
obtaining a clarified fermentation broth from growth of a microorganism to produce at least one diammonium salt of a C4 diacid selected from the group consisting of succinic, malic, maleic and fumaric acid and wherein the clarified fermentation broth containing the ammonium salt of a C4 diacid Is obtained by:
- growing a microorganism In a fermentation media to produce a fermentation broth containing the C4 diacid;
- contacting the fermentation broth with the C4 diacid with a calcium compound to form a calcium salt of the diacid;
- contacting the fermentation broth with a source of ammonium and with a source of carbonate under a first condition including a temperature of at least 100°C and a pressure of at least 1480.28 kPa [200 psig] for a time sufficient to form a carbonate salt of at least 90% of the calcium and an ammonium salt of at least 90% of the C4 diacid in the fermentation broth;
- reducing the temperature and pressure of the contacted fermentation broth to a second condition effective to form a precipitate of the carbonate salt of calcium while maintaining the ammonium salt of the diacid in solution; and
- separating the precipitated carbonate salt of the calcium and cell mass in the fermentation broth from the solution of the ammonium salt of the diacid to form the clarified fermentation broth containing the ammonium salt of a C4 diacid; and
contacting the clarified fermentation broth containing the diammonium salt of the C4 diacid with a hydrogenation catalyst and H₂ in a reaction mixture under conditions of temperature, pressure and time sufficient to hydrogenate the diammonium salt of the C4 diacid to form a derivative compound selected from the group consisting of gamma-butyrolactone (GBL) and 1,4-butane-diol (BDO).

2. The method of claim 1 wherein the derivative compound Is GBL; C4 diacids In the fermentation broth comprise primarily succinate or are reduced to succinic acid during the hydrogenation; a dioxane solvent Is included In the hydrogenation reaction mixture; and the hydrogenation catalyst for formation of the GBL comprises palladium and Al₂O₃.

3. The method of claim 1 wherein the ammonium salt of the C4 diacid in the clarified fermentation is primarily ammonium malate, and the clarified fermentation broth is contacted with an acid catalyst at a temperature sufficient to dehydrate malate into fumarate in the reaction mixture.

4. The method of claim 1 wherein the C4 diacid Is primarily at least one of ammonium fumarate and ammonium malate and including steps wherein:
the clarified fermentation broth Is contacted with an acid catalyst and methanol forming an intermediate selected from the group consisting of dimethyl fumarate and/or dimethyl malate; and
ammonium is separated from the dimethyl fumarate and/or dimethyl malate prior to contacting with the hydrogenation catalyst; and
the C4 derivative compound Is BDO.

5. The method of claim 4 wherein the acid catalyst is a homogenous acid.

6. The method of claim 4 wherein the acid catalyst is a heterogeneous acidic resin.

7. The method of claim 1 wherein the ammonium salt of the C4 diacid in the clarified fermentation broth is primarily ammonium succinate, the
ammonium is removed from the clarified fermentation broth forming free succinic acid prior to contacting with the hydrogenation catalyst and the derivative compound is BDO.

8. The method of claim 1 wherein the hydrogenation catalyst is a compound composing at least one of nickel, ruthenium, rhodium, palladium, platinum and copper chromite.

9. The method of claim 8 wherein the diammonium salt of the C4 diacids in the clarified fermentation is primarily at least one of ammonium fumarate and ammonium malate, and contacting with the hydrogenation catalyst includes contacting with a first catalyst comprising nickel and a second catalyst comprising at least one of ruthenium, rhodium and palladium.

10. The method of claim 9 wherein contacting with the first catalyst is done prior to contacting with the second catalyst.

11. The method of claim 1 wherein the diammonium salt of the C4 diacids in the clarified fermentation is primarily at least one of ammonium fumarate and ammonium malate and the contacting with the hydrogenation catalyst and hydrogen includes: (i) a first hydrogenation step wherein the contacting is for a time sufficient to hydrogenate the fumarate and/or malate to form succinate; (ii) an intervening step wherein the ammonium is replaced with hydrogen forming free succinic acid; and (iii) a second hydrogenation step wherein the contacting is for a time sufficient to form BDO from the succinic acid.

12. The method of claim 11 wherein the intervening step is selected from the group consisting of ion exchange chromatography, electrodialysis, and electro deionization.

13. The method of claim 1 wherein the C4 diacid is primarily at least one of ammonium fumarate and ammonium malate; the clarified fermentation broth is contacted with an acid catalyst and methanol forming at least one of dimethyl fumarate and dimethyl malate and prior to contacting with the hydrogenation catalyst an intervening step is included wherein ammonium is removed from the reaction mixture; and wherein contacting with the hydrogenation catalyst is for a time sufficient to form BDO from at least one of the dimethyl fumarate and dimethyl malate.

14. The method of claim 13 wherein the intervening step is selected from the group consisting of Ion exchange chromatography, electrodialysis, and electro deionization.

15. The method of claim 1 wherein the wherein the source of ammonium comprises NH₄OH.

16. The method of claim 1 wherein the calcium compound is In a form selected from the group consisting of an oxide, a hydroxide, a carbonate and a bicarbonate of the calcium.

17. The method of claim 16 wherein the calcium compound is introduced Into the fermentation media to maintain the pH of the fermentation media between a pH of 5.5 to 7.5 during a period of production of the C4 diacid by the microorganism.

18. The method of claim 17 wherein the calcium compound is obtained by separating a precipitated carbonate salt of calcium from a second fermentation media used to produce the C4 diacid.

## Patentansprüche

1. Verfahren zur Herstellung einer C4-Säurederivatverbindung, umfassend:
Gewinnen einer geklärten Fermentationsbrühe aus dem Wachstum eines Mikroorganismus unter Produktion von wenigstens einem Diammoniumsalz einer C4-Disäure, die aus der Gruppe ausgewählt ist, die aus Bernstein-, Äpfel-, Malein- und Fumarsäure besteht, wobei die geklärte Fermentationsbrühe, die das Ammoniumsalz einer C4-Disäure enthält, erhalten wird durch:
- Wachsenlassen eines Mikroorganismus in einem Fermentationsmedium unter Bildung einer Fermentationsbrühe, die die C4-Disäure enthält;
- In-Kontakt-Bringen der Fermentationsbrühe, die die C4-Disäure enthält, mit einer Calciumverbindung unter Bildung eines Calciumsalzes der Disäure;
- In-Kontakt-Bringen der Fermentationsbrühe mit einer Ammoniumquelle und mit einer Carbonatquelle unter einer ersten Bedingung, die eine Temperatur von wenigstens 100 °C und einen Druck von wenigstens 1480,28 kPa [200 psig] umfasst, während einer ausreichenden Zeit, um ein Carbonatsalz von wenigstens 90% des Calciums und ein Ammoniumsalz von wenigstens 90% der C4-Disäure in der Fermentationsbrühe zu bilden;
- Reduzieren der Temperatur und des Drucks der in Kontakt gebrachten Fermentationsbrühe auf eine zweite Bedingung, die die Bildung eines Niederschlags des Carbonatsalzes von Calcium bewirkt, während das Ammoniumsalz der Disäure in Lösung gehalten wird; und
- Abtrennen des ausgefällten Carbonatsalzes des Calciums und der Zellmasse in der Fermentationsbrühe von der Lösung des Ammoniumsalzes der Disäure unter Bildung der geklärten Fermentationsbrühe, die das Ammoniumsalz einer C4-Disäure enthält; und
In-Kontakt-Bringen der geklärten Fermentationsbrühe, die das Diammoniumsalz der C4-Disäure enthält, mit einem Hydrierungskatalysator und H₂ in einem Reaktionsgemisch unter Bedingungen von Temperatur, Druck und Zeit, die ausreichen, um das Diammoniumsalz der C4-Disäure zu hydrieren, wobei eine Derivatverbindung entsteht, die aus der Gruppe ausgewählt ist, die aus gamma-Butyrolacton (GBL) und 1,4-Butandiol (BDO) besteht.

2. Verfahren gemäß Anspruch 1, wobei die Derivatverbindung GBL ist, die C4-Disäuren in der Fermentationsbrühe hauptsächlich Succinat umfassen oder während der Hydrierung zu Bernsteinsäure reduziert werden, in dem Hydrierungsreaktionsgemisch ein Dioxan-Lösungsmittel mitverwendet wird und der Hydrierungskatalysator zur Bildung des GBL Palladium und Al₂O₃ umfasst.

3. Verfahren gemäß Anspruch 1, wobei das Ammoniumsalz der C4-Disäure in der geklärten Fermentationsbrühe hauptsächlich Ammoniummalat ist und die geklärte Fermentationsbrühe bei einer ausreichenden Temperatur, um Malat in dem Reaktionsgemisch zu Fumarat zu dehydratisieren, mit einem Säurekatalysator in Kontakt gebracht wird.

4. Verfahren gemäß Anspruch 1, wobei die C4-Disäure hauptsächlich wenigstens eines aus Ammoniumfumarat und Ammoniummalat ist, umfassend Schritte, bei denen:
die geklärte Fermentationsbrühe mit einem Säurekatalysator und Methanol in Kontakt gebracht wird, wobei ein Zwischenprodukt entsteht, das aus der Gruppe ausgewählt ist, die aus Dimethylfumarat und/oder Dimethylmalat besteht; und
Ammonium von dem Dimethylfumarat und/oder Dimethylmalat abgetrennt wird, vor dem In-Kontakt-Bringen mit dem Hydrierungskatalysator; und
die C4-Derivatverbindung BDO ist.

5. Verfahren gemäß Anspruch 4, wobei der Säurekatalysator eine homogene Säure ist.

6. Verfahren gemäß Anspruch 4, wobei der Säurekatalysator ein heterogenes saures Harz ist.

7. Verfahren gemäß Anspruch 1, wobei das Ammoniumsalz der C4-Disäure in der geklärten Fermentationsbrühe hauptsächlich Ammoniumsuccinat ist, das Ammonium unter Bildung von freier Bernsteinsäure aus der geklärten Fermentationsbrühe vor dem In-Kontakt-Bringen mit dem Hydrierungskatalysator entfernt wird und die Derivatverbindung BDO ist.

8. Verfahren gemäß Anspruch 1, wobei der Hydrierungskatalysator eine Verbindung ist, die wenigstens eines aus Nickel, Ruthenium, Rhodium, Palladium, Platin und Kupferchromit enthält.

9. Verfahren gemäß Anspruch 8, wobei das Diammoniumsalz der C4-Disäuren in der geklärten Fermentationsbrühe hauptsächlich wenigstens eines aus Ammoniumfumarat und Ammoniummalat ist und das In-Kontakt-Bringen mit dem Hydrierungskatalysator ein In-Kontakt-Bringen mit einem ersten Katalysator, der Nickel umfasst, und einem zweiten Katalysator, der wenigstens eines aus Ruthenium, Rhodium und Palladium umfasst, umfasst.

10. Verfahren gemäß Anspruch 9, wobei das In-Kontakt-Bringen mit dem ersten Katalysator vor dem In-Kontakt-Bringen mit dem zweiten Katalysator erfolgt.

11. Verfahren gemäß Anspruch 1, wobei das Diammoniumsalz der C4-Disäuren in der geklärten Fermentationsbrühe hauptsächlich wenigstens eines aus Ammoniumfumarat und Ammoniummalat ist und das In-Kontakt-Bringen mit dem Hydrierungskatalysator und Wasserstoff umfasst: (i) einen ersten Hydrierungsschritt, bei dem das In-Kontakt-Bringen während einer ausreichenden Zeit erfolgt, um das Fumarat und/oder Malat unter Bildung von Succinat zu hydrieren; (ii) einen Zwischenschritt, bei dem das Ammonium durch Wasserstoff ersetzt wird, wobei freie Bernsteinsäure entsteht; und (iii) einen zweiten Hydrierungsschritt, bei dem das In-Kontakt-Bringen während einer ausreichenden Zeit erfolgt, um aus der Bernsteinsäure BDO zu bilden.

12. Verfahren gemäß Anspruch 11, wobei der Zwischenschritt aus der Gruppe ausgewählt ist, die aus Ionenaustauschchromatographie, Elektrodialyse und Elektrodeionisierung besteht.

13. Verfahren gemäß Anspruch 1, wobei die C4-Disäure hauptsächlich wenigstens eines aus Ammoniumfumarat und Ammoniummalat ist; die geklärte Fermentationsbrühe mit einem Säurekatalysator und Methanol in Kontakt gebracht wird, wobei wenigstens eines aus Dimethylfumarat und Dimethylmalat entsteht, und vor dem In-Kontakt-Bringen mit dem Hydrierungskatalysator ein Zwischenschritt eingeschaltet wird, bei dem Ammonium aus dem Reaktionsgemisch entfernt wird; und wobei das In-Kontakt-Bringen mit dem Hydrierungskatalysator während einer ausreichenden Zeit erfolgt, um aus wenigstens einem aus Dimethylfumarat und Dimethylmalat BDO zu bilden.

14. Verfahren gemäß Anspruch 13, wobei der Zwischenschritt aus der Gruppe ausgewählt ist, die aus Ionenaustauschchromatographie, Elektrodialyse und Elektrodeionisierung besteht.

15. Verfahren gemäß Anspruch 1, wobei die Ammoniumquelle NH₄OH umfasst.

16. Verfahren gemäß Anspruch 1, wobei die Calciumverbindung in einer Form vorliegt, die aus der Gruppe ausgewählt ist, die aus einem Oxid, einem Hydroxid, einem Carbonat und einem Hydrogencarbonat des Calciums besteht.

17. Verfahren gemäß Anspruch 16, wobei die Calciumverbindung in das Fermentationsmedium eingeführt wird, um den pH-Wert des Fermentationsmediums während der Zeit der Produktion der C4-Disäure durch den Mikroorganismus zwischen pH 5,5 und 7,5 zu halten.

18. Verfahren gemäß Anspruch 17, wobei die Calciumverbindung dadurch erhalten wird, dass man ein ausgefälltes Carbonatsalz von Calcium von einem zweiten Fermentationsmedium, das zur Produktion der C4-Disäure verwendet wird, abtrennt.

## Revendications

1. Procédé de préparation d'un composé dérivé d'un acide en C4, comprenant :
l'obtention d'un bouillon de fermentation clarifié par croissance d'un microorganisme pour produire au moins un sel de diammonium d'un diacide en C4 choisi dans le groupe consistant en l'acide succinique, malique, maléique et fumarique, et dans lequel le bouillon de fermentation clarifié contenant le sel d'ammonium d'un diacide en C4 est obtenu par :
- croissance d'un microorganisme dans un milieu de fermentation pour produire un bouillon de fermentation contenant le diacide en C4 ;
- mise en contact du bouillon de fermentation contenant le diacide en C4 avec un composé du calcium pour former un sel de calcium du diacide ;
- mise en contact du bouillon de fermentation avec une source d'ammonium et avec une source de carbonate dans une première condition comprenant une température d'au moins 100 °C et une pression d'au moins 1480,28 kPa [200 psig] pendant un temps suffisant pour former un sel de carbonate d'au moins 90 % du calcium et un sel d'ammonium d'au moins 90 % du diacide en C4 dans le bouillon de fermentation ;
- réduction de la température et de la pression du bouillon de fermentation mis en contact, jusqu'à une deuxième condition, efficace pour former un précipité du sel de carbonate du calcium tout en maintenant en solution le sel de diammonium dudit acide ; et
- séparation, d'avec la solution du sel d'ammonium du diacide, du sel de carbonate du calcium précipité et de la masse cellulaire se trouvant dans le bouillon de fermentation, pour former le bouillon de fermentation clarifié contenant le sel d'ammonium d'un diacide en C4 ; et
la mise en contact du bouillon de fermentation clarifié, contenant le sel de diammonium du diacide en C4, avec un catalyseur d'hydrogénation et du H₂ dans un mélange réactionnel dans des conditions de température, de pression et de temps suffisantes pour hydrogéner le sel de diammonium du diacide en C4 dans le but de former un composé dérivé choisi dans le groupe consistant en la gamma-butyrolactone (GBL) et le 1,4-butane-diol (BDO).

2. Procédé selon la revendication 1, dans lequel le composé dérivé est la GBL ; les diacides en C4 se trouvant dans le bouillon de fermentation comprennent essentiellement un succinate ou sont réduits en acide succinique pendant l'hydrogénation ; un solvant de type dioxanne est incorporé dans le mélange réactionnel d'hydrogénation ; et le catalyseur d'hydrogénation, pour la formation de la GBL, comprend du palladium et de l'Al₂O₃.

3. Procédé selon la revendication 1, dans lequel le sel d'ammonium du diacide en C4 se trouvant dans le bouillon de fermentation clarifié est essentiellement le malate d'ammonium, et le bouillon de fermentation clarifié est mis en contact avec un catalyseur acide à une température suffisante pour déshydrater le malate en fumarate dans le mélange réactionnel.

4. Procédé selon la revendication 1, dans lequel le diacide en C4 est essentiellement le fumarate d'ammonium et/ou le malate d'ammonium, et comprenant les étapes dans lesquelles :
le bouillon de fermentation clarifié est mis en contact avec un catalyseur acide et du méthanol, formant un intermédiaire choisi dans le groupe consistant en le fumarate de diméthyle et/ou la malate de diméthyle ; et
l'ammonium est séparé du fumarate de diméthyle et/ou du malate de diméthyle avant mise en contact avec le catalyseur d'hydrogénation ; et
le composé dérivé en C4 est le BDO.

5. Procédé selon la revendication 4, dans lequel le catalyseur acide est un acide homogène.

6. Procédé selon la revendication 4, dans lequel le catalyseur acide est une résine acide hétérogène.

7. Procédé selon la revendication 1, dans lequel le sel d'ammonium du diacide en C4 se trouvant dans le bouillon de fermentation clarifié est essentiellement le succinate d'ammonium, l'ammonium est éliminé du bouillon de fermentation clarifié pour former de l'acide succinique libre avant mise en contact avec le catalyseur d'hydrogénation, et le composé dérivé est le BDO.

8. Procédé selon la revendication 1, dans lequel le catalyseur d'hydrogénation est un composé comprenant du nickel, ruthénium, rhodium, palladium, platine et/ou chromite de cuivre.

9. Procédé selon la revendication 8, dans lequel le sel de diammonium des diacides en C4 se trouvant dans le bouillon de fermentation clarifié est essentiellement le fumarate d'ammonium et/ou le malate d'ammonium, et la mise en contact avec le catalyseur d'hydrogénation comprend une mise en contact avec un premier catalyseur comprenant du nickel et un deuxième catalyseur comprenant du ruthénium, du rhodium et/ou du palladium.

10. Procédé selon la revendication 9, dans lequel la mise en contact avec le premier catalyseur est effectuée avant la mise en contact avec le deuxième catalyseur.

11. Procédé selon la revendication 1, dans lequel le sel de diammonium des diacides en C4 se trouvant dans le bouillon de fermentation clarifié est essentiellement le fumarate d'ammonium et/ou le malate d'ammonium, et la mise en contact avec le catalyseur d'hydrogénation et l'hydrogène comprend : (i) une première étape d'hydrogénation, dans laquelle la mise en contact se fait pendant un temps suffisant pour hydrogéner le fumarate et/ou le malate dans le but de former du succinate ; (ii) une étape intermédiaire, dans laquelle l'ammonium est remplacé par de l'hydrogène, dans le but de former de l'acide succinique libre ; et (iii) une deuxième étape d'hydrogénation, où la mise en contact s'effectue pendant un temps suffisant pour former du BDO à partir de l'acide succinique.

12. Procédé selon la revendication 11, dans lequel l'étape intermédiaire est choisie dans le groupe consistant en la chromatographie par échange d'ions, l'électrodialyse et l'électrodésionisation.

13. Procédé selon la revendication 1, dans lequel le diacide en C4 est essentiellement le fumarate d'ammonium et/ou le malate d'ammonium ; le bouillon de fermentation clarifié est mis en contact avec un catalyseur acide et du méthanol pour former du fumarate de diméthyle et/ou du malate de diméthyle et, avant la mise en contact avec le catalyseur d'hydrogénation, une étape intermédiaire est prévue, dans laquelle l'ammonium est éliminé du mélange réactionnel ; et dans lequel la mise en contact avec le catalyseur d'hydrogénation s'effectue pendant un temps suffisant pour former du BDO à partir du fumarate de diméthyle et/ou du malate de diméthyle.

14. Procédé selon la revendication 13, dans lequel l'étape intermédiaire est choisie dans le groupe consistant en la chromatographie par échange d'ions, l'électrodialyse et l'électrodésionisation.

15. Procédé selon la revendication 1, dans lequel la source d'ammonium comprend du NH₄OH.

16. Procédé selon la revendication 1, dans lequel le composé du calcium se présente sous une forme choisie dans le groupe consistant en un oxyde, un hydroxyde, un carbonate et un bicarbonate du calcium.

17. Procédé selon la revendication 16, dans lequel le composé du calcium est introduit dans le milieu de fermentation pour maintenir le pH du milieu de fermentation entre pH 5,5 et 7,5 pendant une période de production du diacide en C4 par le microorganisme.

18. Procédé selon la revendication 17, dans lequel le composé du calcium est obtenu par séparation, d'avec un deuxième milieu de fermentation utilisé pour produire le diacide en C4, d'un sel carbonate de calcium précipité.
